# EUROPÄISCHE PATENTSCHRIFT

(11) **EP 0 842 405 B1**
(45) Veröffentlichungstag und Bekanntmachung des Hinweises auf die Patenterteilung: **01.08.2001**
(21) Anmeldenummer: 96919648.4
(22) Anmeldetag: 02.05.1996
(51) Int. Cl.: G01L 1/24

(54) **TAKTILER, OPTOELEKTRONISCHER DRUCKSENSOR**
TACTILE OPTO-ELECTRONIC PRESSURE SENSOR
CAPTEUR DE PRESSION TACTILE OPTOELECTRONIQUE

(30) Priorität: 29.07.1995 DE 19527957
(43) Veröffentlichungstag der Anmeldung: 20.05.1998
(73) Patentinhaber: Forschungszentrum Karlsruhe GmbH, 76133 Karlsruhe (DE)
(72) Erfinder: WOLF, Rainer, D-96484 Gro walbur (DE); GAMER, Lothar, D-76646 Bruchsal (DE); FISCHER, Harald, D-76185 Karlsruhe (DE)
(74) Vertreter: Rückert, Friedrich, Dr.
(86) Internationale Anmeldenummer: EP9601810
(87) Internationale Veröffentlichungsnummer: WO9705461

(56) Entgegenhaltungen:
- DE-A- 3 142 301
- US-A- 4 599 908
- US-A- 4 814 562

## Beschreibung

Die Erfindung betrifft ein System, bestehend aus einem taktilen Drucksensor mit einem lichtundurchlässigen, zylindrischen Körper (1), der aus einem Elastomer besteht, mehrere, axial verlaufende Bohrungen (2) aufweist, und einer Bilderfassungseinrichtung mit Datenverarbeitungsanlage. Der Drucksensor beantwortet die Druckausübung auf seinen taktilen Teil mit einer Lichtintensitätsänderung. Diese Änderung der Lichtintensität wird über eine Lichtübertragungsstrecke auf eine Bilderfassungseinrichtung gelenkt und aufgenommen. Eine an die Bilderfassungseinrichtung angeschlossene Datenverarbeitungsanlage steuert diese und liest die intensitätsrelevanten Daten aus. Die Druckeinwirkung auf die drucksensitive Fläche läßt sich dann darstellen, oder es können entsprechende Steuerbefehle an angeschlossene Bedieneinrichtungen oder Empfindungseinrichtungen geleitet werden.

Solche Drucksensoren können für vielerlei Zwecke eingesetzt werden. So kommen sie als Tasteinrichtung in der Robotertechnik in Frage. Es kann eine Oberfläche mit ihnen ertastet werden. In der Medizin können mit solchen Sensoren Gewebepartien oder Organe betastet werden.

Das Druckarray der Firma Interlink wird z.B. in einer laparoskopischen Faßzange verwendet, um die Druckdaten zu erfassen. Der Sensor basiert auf einem halbleitenden Polymer, das bei Beaufschlagung von äußerem Druck seinen Widerstand ändert. Diese nur für dynamische Druckerfassung geeigneten Druckdaten werden nach graphischer Darstellung auf einem PC-Monitor wieder auf ein taktiles System zurückgegeben (siehe Fischer, H. et al., "Messungen der Greifkräfte chirurgischer Zangen mit FSRTM-Sensoren", Interner Bericht, Forschungszentrum KA, HIT, 1994). Das halbleitende Polymer hat die nachteilige Eigenschaft, daß es bei häufiger Betätigung einer raschen Alterung unterliegt, mit sehr hohem Kriechverhalten behaftet ist und somit zur statischen Druckmessung ungeeignet ist, wodurch dann der Rückschluß auf den ausgeübten Druck falsch wird.

Sehr verbreitet sind taktile kapazitive Foliensensoren. Diese bestehen meist aus zwei gekreuzt aufeinandergelegten Kupferstreifen, die durch ein dielektrisches Elastomer voneinander getrennt sind und so eine Matrix aus Kondensatoren bilden (siehe J. Seekircher et al. "Improved tactile sensors", selected papersfrom the 2nd IFAC symposium, Pergamon 1989, p. 317-22). Der Druck auf den Sensor wird in eine Kapazitätsänderung transformiert. Eine solche Einrichtung ist gegen elektromagnetische Felder sehr störanfällig, da die Signalgröße aufgrund der sehr geringen Kapazitätsänderung von 0.6pF bei d/d = 10% und bei einer Schichtdicke von 30*µ*m sehr gering ist und somit aufwendige Auswerteverfahren nötig sind.

In der DE 32 36 435 C2 wird ein faseroptischer Sensor beschrieben, bei dem Licht durch ein Rohr geleitet wird, dessen lichte Weite druckabhängig ist und die Intensität des zu einem Lichtempfänger gelangenden Lichts bestimmt. Mit diesem Sensor kann allerdings vom Prinzip her kein Array aus einer Schicht aufgebaut werden.

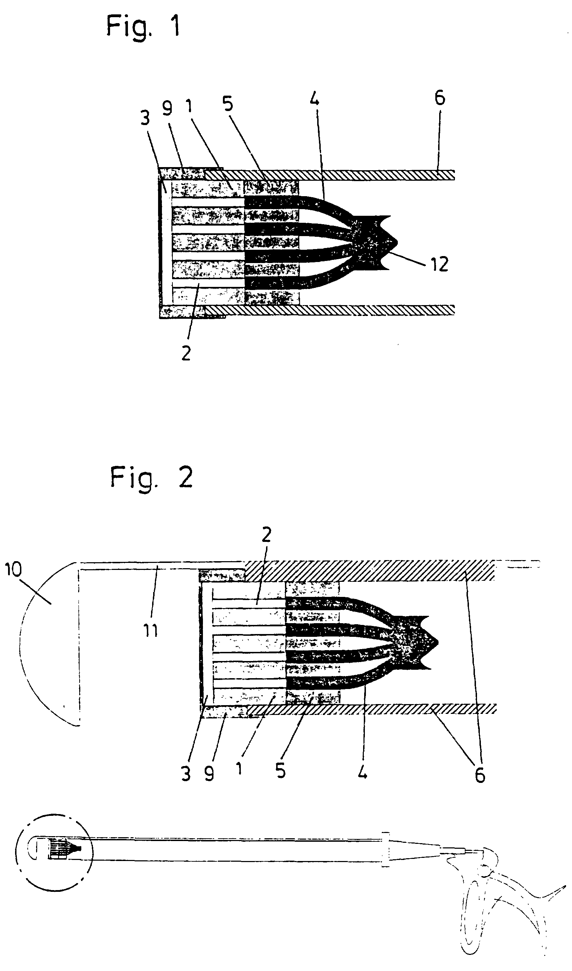

Ein Druck-Intensitäts-Übertragungsprinzip, das den Bau taktiler Sensoren mit kleiner Bauweise und hoher Auflösung für den Einbau in den Finger eines Robotergreifers ermöglicht, wird in ROBOT SENSORS Volume 2, Tactile and Non-Vision, edited by Prof. A. Pugh, Springer-Verlag Berlin Heidelberg New York Tokyo, 1986 in dem Beitrag "An Experimental Very High Resolution Tactile Sensor Array" auf den Seiten 179 bis 188 von D.H. Mott et al. beschrieben. Figur 1 auf Seite 181 zeigt das Prinzip der Umsetzung der Druck-Intensität in Licht-Intensität.

Der taktile Teil besteht aus einer durchsichtigen Akryl Platte, auf die, durch ein Luftkissen getrennt, eine Folie in Form einer nachgiebigen Membran gelegt ist. In die Akryl-Platte wird seitlich Licht eingestrahlt, das im unbelasteten Zustand durch die Platte geht, an den Seitenwänden reflektiert wird und an der gegenüberliegenden Stirnseite austritt. Wird die Membran jedoch durch äußere Druckeinwirkung an irgend einer Stelle auf die Akryl-Platte gedrückt, dann kommt es dort zu einer diffusen Reflektion aufgrund der dort geänderten Verhältnisse im Brechungsindex und Licht strahlt von dort aus durch die Seitenwände.

Das Bild des vom taktilen Sensor diffus reflektierten Lichts wird über optische Einrichtung auf die Eintrittsfläche eines CCD-Chips mit vorgeschalteter Optik gelenkt und in einer angeschlossenen Datenverarbeitungsanlage zur Darstellung der Videosignale/-bilder oder zur Erzeugung von Steuersignalen für den Robotergreifer weiterverarbeitet. Des weiteren wird die CCD-Camera von der Datenverarbeitungsanlage gesteuert und betrieben. Es werden ausschließlich Videobilder erzeugt, die durch eine notwendigerweise vorgeschaltete Linsenoptik erzeugt wird. Eine Aussage über die aufgebrachte Kraft kann mit diesem System nicht gemacht werden.

Die mit CCD-Chips in der Regel erzeugten Video-Bilder müssen in diesem Zusammenhang hinterher durch aufwendige Bilderkennungsverfahren ausgewertet werden, da wegen der beispielsweise 486 923 Pixel riesige Datenmengen vorliegen. Bei Verwendung von PC-Auswerteeinheiten können riesige Datenmengen, wie sie beim Verschieben in seriellen Schnittstellen vorkommen, zwar abgearbeitet werden. Durch die bei der CCIR-Norm (internationale Fernsehnorm) verwendete Hardware erfolgt keinerlei Datenreduktion. Bei späterer Auswertung muß dies durch aufwendige Software-Programme erfolgen. Das Gesamtsystem ist als Video-Einheit schnell, jedoch erfordert die Datenauswertung zwischen der Intensitätsmessung der Lichtverteilung auf dem CCD-Chip sehr viel Zeit. Standard CCD-Auswertungen liefern nur visuelle Bilder ohne Zuordnung des auf dem Sensor aufgebrachten Drucks bzw. der Lichtintensität als Spannungssignal. Sie dienen in der Regel zur visuellen Erkennung von Gegenständen (Texturen, Umrandungen).

In der US-4,599,908 wird ein Drucksensor mit einem zylindrischen Körper aus Elastomer beschrieben, der mehrere, axial verlaufende Bohrungen aufweist. Bei diesem Sensor wird das Licht über Wellenleiter auf den Sensor eingekoppelt und am druckempfindlichen Ende des Sensors druckabhängig reflektiert.

Der Erfindung liegt die Aufgabe zugrunde, statische als auch dynamische Druckverteilungen an Körpern oder Gegenständen mithoher Auflösung auf kleiner Fläche absolut und dynamisch zu messen. Hierzu soll ein System, bestehend aus einem Drucksensor und Bilderfassungseinrichtung mit Datenverarbeitungsanlage entwickelt werden. Mit diesem System soll das "Fingerspitzengefühl" graphisch auf einem Monitor dargestellt werden können. Ebenso soll ein solcher System zur Ansteuerung von Aktoren geeignet sein.

Die Aufgabe wird erfindungsgemäß durch ein System gemäß den Merkmalen des Anspruchs 1 gelöst.

Im Anspruch 2 sind verschiedene vorteilhafte Bauformen für die Lichtübertragungsstrecke gekennzeichnet. Anspruch 3 kennzeichnet eine bauliche Schutzmaßnahme, die optoelektronische Schnittstelle von äußeren Einflüssen abschottet.

Die beiden Ansprüche 4 und 5 kennzeichnen Bauformen des optoelektronischen Sensors, die für die eine oder andere Einsatzart zweckmäßig ist.

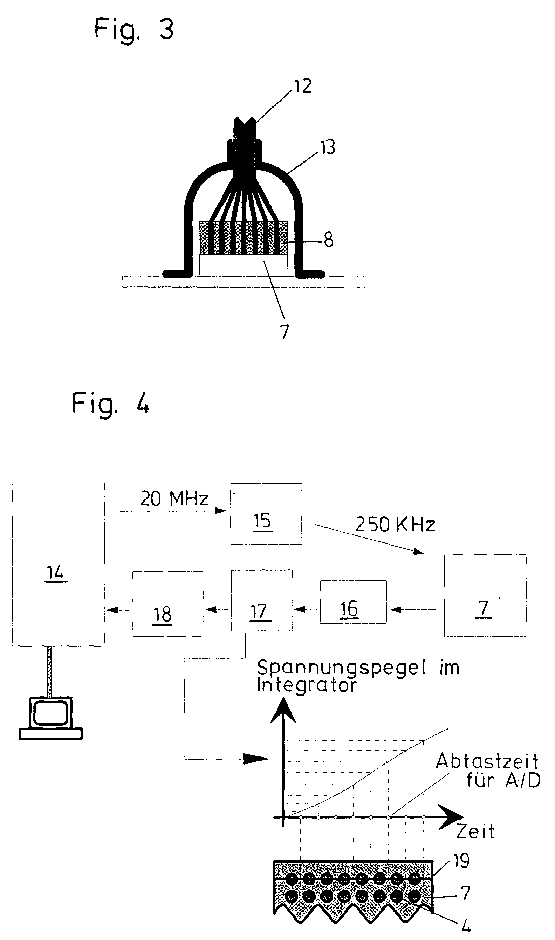

Um die Datenflut beim Kommunizieren mit dem CCD-Chip optimal und zeitsparend zu beherrschen, wurde ein darauf abgestimmtes Signal- und Datenverarbeitungssystem eingesetzt, das im Anspruch 6 gekennzeichnet ist. Figur 4 der Zeichnung zeigt im Blockschaltbild das Zusammenwirken der Systemblöcke.

Anspruch 7 kennzeichnet eine ganz spezifisch zugeschnittene Hardware-Konfiguration, mit der das CCD-Chip getaktet wird.

Eine nachlassende physikalische Eigenschaft wie bei halbleitenden Polymeren ist bei Elastomeren, EL's und Lichtwellenleitern nicht zu erwarten. Durch die geringe Anzahl von Meßdaten, die der Mikroprozessor über die serielle Schnittstelle zur Verfügung stellt, kann im unbelasteten Zustand des taktilen Sensors die Vorkalibrierung (Referenzmessung) erfolgen. Hierzu wird das von der elektroluminiszenten Folie erzeugte Licht, das noch an der andern Seite des perforierten Gummikörpers austritt, in seiner Intensität gemessen. Damit kann eigentlich nach jeder vollständigen Entlastung des perforierten Körperseine Leerlaufkalibrierung erfolgen, d. h. höchste Abstrahlung an der Lichtaustrittsfläche wird der Nullbelastung des elastomeren, zylindrischen Körpers gleichgesetzt. Ein senkrecht ausgeübter Druck auf die mit der elektroluminiszenten Folie abgedeckten Seite verkleinert die lichten Querschnitte der Bohrungen bis hin zum vollen Verschluß, womit dann die oberste, quantitativ erfaßbare Druckbelastung auf den Elastomer erreicht wäre. Der Meßbereich kann erweitert oder verringert werden, indem ein Elastomer mit entsprechendem Elastizitätsmodul ausgewählt wird.

Der Vorteil mit dem Signal- und Datenverarbeitungssystem gegenüber der üblichen Auswertung von CCD-Chips liegt in der ganz enormen Datenreduzierung des steuernden und treibenden Mikroprozessors um den Faktor 817x596 = 486 923 Pixel/64 digitale Meßwerte nach Zusammenfassung der entsprechenden Zeilen. Die Zahlenangaben betreffen hier einen ganz bestimmten CCD-Typ. Für andere Anwendungen kann ein anderer CCD-Typ mit mehr oder weniger Pixel auf der lichtempfindlichen Eintrittsfläche interessant sein. Das wird erreicht: einerseits durch die Datenreduktion über das Konzept der Zeilenintegration und andererseits durch die softwaremäßige Zusammenfassung der Meßwerte zu 64 Clustern durch einen schnellen Rechenalgorithmus im Mikroprozessor. Würde immer nur ein Halbbild ausgelesen werden, könnte eine weitere Datenreduzierung um den Faktor 2 erfolgen.

Der entscheidende Vorteil liegt aber in der Zeit, man benötigt dann nur noch die Hälfte davon.

Das liegt daran, daß im Unterschied zur Video-Auswertung von CCD-Chips keine optische Objekterkennung stattfindet, sondern nur eine Aussage über einen örtlichen verteilten Druck durch entsprechend örtlich verteilte Lichtintensität gemacht werden soll. Es sind also nur bestimmte Pixel, nämlich die lichtbeschienen von Interesse, die stets dunkelbleibenden interessieren nicht. Zudem ist durch dieses Meßverfahren ein sonst übliches, zusätzliches Linsensystem als Vorschaltoptik nicht mehr notwendig und kann daher entfallen.
Die so gewonnenen Daten können zur Darstellung auf einem Monitor in Form eines Druckgebirges verwendet werden oder zur Ansteuerung eines taktilen Arrays, das das Druckprofil auf die Fingerspitzen des Bedieners überträgt. Mit diesem optoelektronischen Drucksensor bekommt der Betreiber sein Gefühl wieder zurück, obwohl er den Gegenstand nicht unmittelbar betastet.

Der erfindungsgemäß aufgebaute taktile Sensor des optoelektronischen Sensors hat den Vorteil in seiner Lichteinstrahlung durch die flexible, elektroluminiszente Folie. Das zu betastende Objekt kann durch etwaige Schattenbildung das Meßergebnis nicht beeinflussen. Insgesamt ist der optoelektronische Drucksensor durch seinen Aufbau gegen äußere Verschmutzung lückenlos abgeschirmt.

Ein Ausführungsbeispiel der Erfindung ist in der Zeichnung schematisch dargestellt und wird im folgenden näher beschrieben. Es zeigt:
Figur 1 den taktilen Sensor,
Figur 2 den taktilen Sensor in eine Zange eingebaut,
Figur 3 die optoelektronische Schnittstelle und
Figur 4 das Blockschaltbild zur Signal- und Datenverarbeitung.

Das im im folgenden beschriebene System ist für den Einsatz in der minimal invasiven Chirurgie konzipiert. Der taktile Drucksensor soll am Ende einer endoskopisch eingesetzten, laparoskopischen Faßzange zur palpatorischen Untersuchung von Gewebe eingebaut werden. Der Chirurg kann sein Fingerspitzengefühl beim Verwenden der heutigen Greifzange nicht mehr einsetzen, weil taktile Empfindungen über den mechanischen Aufbau der Instrumente nicht empfindlich genug übertragen werden. Er versucht deswegen unterbewußt, über die Rezeptoren der Gelenke und Muskeln Information über das zu untersuchende Gewebe zu bekommen. Das sind aber dann mehr oder weniger Lageempfindungen. Zudem erfolgt eine visuelle Kontrolle. Hierbei drückt er das Gewebe bis es weißlich wird und liegt somit immer am oberen Grenzbereich.

Mit einem System, dessen taktiler Teil in das Maulteil eines zangenförmigen Instruments eingebaut ist, wird eine reproduzierbare und kalibrierte Meßeinheit zur direkten Messung von statischen und dynamischen Druckprofilen auf sehr kleiner Fläche bereitgestellt. Darüber hinaus ist mit diesem System Absolutdruckmessung jederzeit möglich.

Das distale Ende einer Faßzange für den endoskopischen Gebrauch ist in der Regel im Durchmesser nicht größer als 10mm. Figur 1 zeigt die schematische Darstellung des taktilen Drucksensors, Figur 2 den Einbau desselben in die Faßzange, deren bewegliches Maulteil 10 für paralleles Greifen konstruiert ist, und zwar so, daß auf den taktilen Teil nur senkrecht in Richtung der Bohrungen 2 gedrückt werden kann. Kraftbegrenzung im Griff der Zange sorgt für eine Vorkalibrierung durch stets gleichmäßiges und definiertes Zusammendrücken der Maulteile.

Der taktile Teil des Drucksensors besteht aus dem zylinderförmigen, lichtundurchlässigen Elastomer 1 (Silikonkautschuk) mit der symmetrischen Anordnung von 64 Bohrungen 2. Die Bohrungen 2 sind gleich und haben je eine lichte Weite von 0,35mm. Die Bohrungen 2 sind Kollimatorbohrungen, die nur senkrechten Lichtdurchgang erlauben. In den Figuren 1 und 2 sind der Übersicht halber nur wenige Bohrungen 2 angedeutet.

Auf der oberen Seite des Elastomers 1 (Figur 1) liegt die flexible, elektroluminiszente Folie 3 unmittelbar auf. Sie ist 0.17mm dick und liegt mitsamt dem freien Ende des Elastomers 1 unter der weichen Umhüllung 9 aus ebenfalls Silikonkautschuk. Die elektrischen Anschlüsse an der Folie 3 sind nicht eingezeichnet. Bei Anlegen einer Spannung leuchtet die Folie die Bohrungen 2 aus und strahlt durch diese hindurch in die unmittelbar an der andern Seite des Elastomers 1 anliegenden Lichtwellenleiter 4. An jeder Bohrung 2 endet ein Lichtwellenleiter 4, der mindestens den Querschnitt der Bohrung 2, höchstens aber 0.5mm hat. Die Lichtwellenleiterenden sind in dem Haltekörper 5 unverrückbar gefaßt und werden an den Elastomer 1 gedrückt, so daß die Lichteinkopplung unmittelbar erfolgt.

Wird der Elastomer 1 nicht gedrückt, behalten die Bohrungen 2 ihre größte lichte Weite. Es wird dann die höchste Lichtintensität in die Lichtwellenleiter 4 eingekoppelt. Wird er in vorgesehener Richtung gedrückt - nicht seitlich oder schräg - da dann die Bohrungen 2 gekrümmt werden - nimmt der Durchmesser der Bohrungen 2 ab und es wird das an den Bohrungen 2 austretende Licht in seiner Intensität geringer. Der Elastomer 1 ist in dem Schaft 6 so gefaßt, daß er nicht nach außen in radialer Richtung ausweichen kann, also wird die Volumenänderung auf Kosten der lichten Weite der Bohrungen 2 kompensiert. Die Druckeinwirkung kann quantitativ nur solange erfaßt werden, bis der völlige optische Verschluß der Bohrungen 2 durch die Druckeinwirkung einsetzt.

Figur 3 zeigt schematisch, wie die Lichtwellenleiter 4 auf dem CCD-Chip 7 enden. Hierzu sind diese Enden wiederum in dem Haltekörper 8 gefaßt und liegen damit unmittelbar auf der Lichteintrittfläche des CCD-Chips 7 an. Wesentlich dabei ist lediglich, daß die Lichteintrittsfläche des Bündels 12 aus Lichtleitern 4 ähnlich der Lichtaustrittsfläche ist und die Enden entsprechend verteilt sind, da dann erst eine örtliche Zuordnung der Lichtintensität zum einwirkenden Druck hergestellt werden kann. Gegen Fremdeinflüsse ist diese optoelektronische Schnittstelle durch ein hermetisch abschließendes Gehäuse 13 abgeschottet. Das Bündel 12 ist flexibel, so daß die optoelektronische Schnittstelle in Form des CCD-Chips von der Zange fern gehalten werden kann.

Das CCD-Chip wird modifiziert betrieben (nicht nach CCIR=intern. Fernsehnorm), wie in Figur 4 schematisch dargestellt. Das für diesen Einsatz entwickelte Auswerteverfahrenbetrifft im wesentlichen die Datenreduzierung und die Verarbeitungsgeschwindigkeit der vom CCD-Chip 7 ausgelesenen Signale.

Das Zentrum der Auswertung stellt der Mikrocontroller dar, der eine maximale Pixel-Frequenz von 250kHz zum Ansteuern des CCD-Chips 7 ermöglicht. Der gesamte Zeitablauf ist softwaremäßig gesteuert und damit hier noch sehr flexibel gestaltet. Die Taktimpulse werden durch die externe Treiberstufe 15 im Pegel umgesetzt.

Die im CCD-Chip 7 erzeugten Ladungsmengen werden in einer nachfolgenden FET-Spannungsstufe in Spannungssignale umgewandelt. Diese stark temperaturabhängigen Sensorsignale werden dann durch das CDS-System 16 im Analogzweig stabilisiert. Es handelt sich dabei um versetzt getaktete, schnelle Sample & Hold-Glieder, die in Verbindung mit einem nachgeschalteten Differenzverstärker Störkomponeneten ausfiltern. Die jetzt stabilisierte Signalspannung gelangt in den Integrator 17, der die einzelnen Signalspannungen aller Pixel aus einer Zeile des CCD-Chip 7 aufsummiert.

Der A/D-Wandler 18 tastet achtmal pro Zeile 19 ab. Durch einfache Differenzbildung ergeben sich die Werte, die der eingespeisten Lichtmenge durch die acht Lichtwellenleiter 4 pro Zeile 19 entsprechen. Damit ist eine Datenreduktion von 817 Pixel-Signalen auf lediglich acht Summen-Signale pro Zeile erfolgt.

Werden jetzt noch alle Zeilen 19 zusammengefaßt, die durch ihre Anordnung im vertikalen Beleuchtungsfeld eines Lichtwellenleiters 4 liegen, erhält man nach Auslesen des kompletten Sensors mit seinen 486 923 Bildpunkten nur die 64 einzelnen Meßwerte, die den flächenmäßig integrierten Lichtmengen der 64 Sensorelemente entsprechen.

### Bezugszeichenliste

- 1: zylindrischer Körper, gummiartiger Körper, Elastomer
- 2: Bohrung
- 3: elektroluminiszente Leuchtfolie
- 4: Lichtwellenleiter, Lichtleitfaser
- 5: Haltekörper
- 6: Rohr, Schaft
- 7: Bilderfassungseinrichtung, CCD-Chip
- 8: Haltekörper
- 9: Umhüllung, Kappe
- 10: bewegliches Maulteil
- 11: Zugstange
- 12: Lichtzuführung, Bündel, Lichtübertragungsstrecke
- 13: Gehäuse
- 14: Mikrocontroller, Mikroprozessor, PC
- 15: Treiberstufe
- 16: CDS-Sytem
- 17: Integrator
- 18: A/D-Wandler
- 19: Zeile

## Patentansprüche

1. System, bestehend aus einem taktilen Drucksensor mit einem lichtundurchlässigen, zylindrischen Körper (1), der aus einem Elastomer besteht, mehrere, axial verlaufende Bohrungen (2) aufweist, und einer Bilderfassungseinrichtung mit Datenverarbeitungsanlage,
dadurch gekennzeichnet, daß
die erste Stirnwandung des Körpers (11) mit einer elektroluminiszenten Leuchtfolie (3), die Licht in die Bohrungen (2) richtet, sowie mit einer weichen Kappe (9) versehen ist, über welcher der zylindrische Körper (1) in seiner axialen Richtung druckbeaufschlagbar ist,
mit einem sich in der axialen Richtung an die weiche Kappe (9) anschließenden, den zylindrischen Körper (1) radial festummantelnden, sich über die zweite Stirnwandung hinaus erstreckenden Rohr (6) und mit einem, die zweite Stirnwandung in der axialen Richtung festlegenden Haltekörper (5), über den das die Bohrungen (2) verlassende Licht der Bilderfassungseinrichtung (7), die eine optoelektronische Schnittstelle hat, zuführbar ist.

2. System nach Anspruch 1,
dadurch gekennzeichnet, daß
das die Bohrungen (2) verlassende Licht über eine Stablinsenoptik oder ein Bildleitfaserbündel oder ein Lichtleitfaserbündel, wobei beim letzteren jede Faser mindestens den Durchmesser der Bohrung (2) hat, der Bilderfassungseinrichtung (7) zugeführt wird und die Bilderfassungseinrichtung ein CCD-Chip ist.

3. System nach Anspruch 2,
dadurch gekennzeichnet, daß
die optoelektronische Schnittstelle, bestehend aus dem Ende der Lichtzuführung (12) und dem CCD-Chip (7), zum Schutz gegen Fremdlicht und Umweltseinflüsse mit einem lichtundurchlässigen Gehäuse (13) umgeben ist.

4. System nach Anspruch 3,
dadurch gekennzeichnet, daß
der taktile Sensor am Ende des Rohrs (6) und ein Teil der Lichtzuführung (12) in dem Rohr (6) gefaßt sind, so daß der Drucksensor als Druckstabsensor verwendet werden kann.

5. System Anspruch 4,
dadurch gekennzeichnet, daß
der Drucksensor in den Schaft (6) eines medizinischen Zangeninstruments eingebaut ist, so daß die druckempfindliche Fläche in Richtung Schaftachse ausgerichtet ist, und ein bewegliches Maulteil (10) mit einer Auflagefläche, die parallel zur druckempfindlichen Fläche liegt, von der druckempfindlichen Fläche weg oder auf sie zu bewegt werden kann, womit dazwischenliegendes Gewebe betastet werden kann.

6. System nach Anspruch 5,
dadurch gekennzeichnet, daß
die Datenverarbeitungsanlage aus einer correlated double sampling (CDS-) Stufe (16), einem nachfolgenden Integrator (17) und einem A/D-Wandler (18) sowie einem Mikroprozessor (14) besteht und die in dem CCD-Chip (7) vorhandenen, ortsabhängigen, lichtintensitätsproportionalen, elektrischen Signale zu einer Druckprofildarstellung verarbeitet, und der Mikroprozessor (14) über eine Treiberstufe (15) das CCD-Chip (7) in vorgegebener Frequenz taktet.

7. System nach Anspruch 6,
dadurch gekennzeichnet, daß
die Treiberstufe (15) aus einer angepaßten Hardware-Konfiguration in ASIC-Technik d.h. Application Specific Integrated Circuit oder FPGA-Technik d.h. Field Programmable Gate Array besteht.

## Claims

1. System, comprising a tactile pressure sensor having a light-impermeable, cylindrical body (1), which comprises an elastomer and has a plurality of axially extending bores (2), and having an image detecting arrangement with a data processing system,
characterised in that
the first end wall of the body (1) is provided with an electroluminescent light-emitting film (3), which directs light into the bores (2), as well as being provided with a soft cap (9), via which the cylindrical body (1) can be applied with pressure in its axial direction,
with a pipe (6), which communicates with the soft cap (9) in the axial direction, firmly surrounds the cylindrical body (1) radially and extends beyond the second end wall, and with a retaining body (5), which secures the second end wall in the axial direction, and via which the light, leaving the bores (2), can be supplied to the image detecting arrangement (7), which has an optoelectronic interface.

2. System according to claim 1, characterised in that the light, leaving the bores (2), is supplied to the image detecting arrangement (7) via a bar lens optical system or a bundle of image carrying fibres or a bundle of optical fibres, each fibre having at least the diameter of the bore (2) in the latter case, and the image detecting arrangement is a CCD chip.

3. System according to claim 2, characterised in that the optoelectronic interface, comprising the end of the light supply means (12) and the CCD chip (7), is surrounded by a light-impermeable housing (13) for protection against extraneous light and environmental influences.

4. System according to claim 3, characterised in that the tactile sensor is gripped at the end of the tube (6), and a portion of the light supply means (12) is gripped in the tube (6), so that the pressure sensor can be employed as a compression bar sensor.

5. System according to claim 4, characterised in that the pressure sensor is incorporated in the shaft (6) of a medical forceps-like instrument so that the pressure-sensitive surface is orientated in the direction towards the shaft axis, and a displaceable mouthpiece (10), with a bearing surface which lies parallel to the pressure-sensitive surface, can be moved away from the pressure-sensitive surface or towards said surface, whereby tissue, lying therebetween, can be scanned.

6. System according to claim 5, characterised in that the data processing system comprises a correlated double sampling (CDS) stage (16), a subsequent integrator (17) and an analog-to-digital converter (18), as well as a microprocessor (14), and it processes the locus-dependent, electrical signals, which are present in the CCD chip (7) and are proportional to the light intensity, to form a pressure profile display, and the microprocessor (14) times the CCD chip (7) in a prescribed frequency via a driver stage (15).

7. System according to claim 6, characterised in that the driver stage (15) comprises an adapted hardware configuration in ASIC technology, i.e. Application Specific Integrated Circuit technology, or FPGA technology, i.e. Field Programmable Gate Array technology.

## Revendications

1. Système formé d'un capteur de pression tactile avec un corps cylindrique (1) opaque, réalisé en élastomère et
ayant plusieurs perçages axiaux (2), et d'une installation de saisie d'image avec une installation de traitement de données,
caractérisé en ce que
- la première paroi frontale du corps (11) est munie d'une feuille électroluminescente (3) qui dirige la lumière dans les perçages (2) ainsi que d'une enveloppe souple (9) par lequel le corps cylindrique (1) est sollicité en pression dans sa direction axiale,
- un tube (6) s'étendant dans la direction axiale, rejoignant l'enveloppe souple (9), enveloppant solidement, radialement le corps cylindrique (1) et s'étendant au-delà de la seconde paroi frontale, et un support (5) qui fixe la seconde paroi frontale dans la direction axiale, et par lequel la lumière sortant des perçages (2) est appliquée à une installation de saisie d'image (7) ayant une interface optoélectronique.

2. Système selon la revendication 1,
caractérisé en ce que
la lumière sortant des perçages (2) est fournie à l'installation de saisie d'image (7) par une optique à lentille en forme de tige ou un faisceau de fibres optiques ou un faisceau de fibres lumineuses, et dans ce cas chaque fibre a au moins le diamètre des perçages (2), et l'installation de saisie d'image est une puce CCD.

3. Système selon la revendication 2,
caractérisé en ce que
l'interface optoélectronique formée de l'extrémité de l'alimentation lumineuse (12) et de la puce (CCD 7), est entourée d'un boîtier (13) opaque pour être protégée contre la lumière extérieure et l'influence de l'environnement.

4. Système selon la revendication 3,
caractérisé en ce que
le capteur tactile à l'extrémité du tube (6) et une partie de l'alimentation lumineuse (12) sont enserrés dans le tube (6) pour utiliser le capteur de pression comme capteur de pression à distance.

5. Système selon la revendication 4,
caractérisé en ce que
le capteur de pression est intégré dans le tube (6) d'un instrument médical à pince pour que la surface sensible à la pression soit alignée en direction de l'axe du corps et qu'un mors mobile (10) avec sa surface d'appui parallèle à la surface sensible à la pression, s'écarte de cette surface sensible à la pression ou se rapproche de celle-ci pour permettre de détecter un tissu interposé.

6. Système selon la revendication 5,
caractérisé en ce que
l'installation de traitement de données se compose d'un étage d'échantillonnage (CDS) double à corrélation (16), d'un intégrateur (17) en aval et d'un convertisseur A/D (18) ainsi que d'un microprocesseur (14), et les signaux électriques, existant dans la puce CCD (7), dépendant du lieu, proportionnels à l'intensité lumineuse, sont traités pour représenter un profil de pression, et le microprocesseur (14) commande l'horloge de la puce CCD (7) selon une fréquence prédéterminée par l'intermédiaire d'un étage pilote (15).

7. Système selon la revendication 6,
caractérisé en ce que
l'étage pilote (15) se compose d'une configuration câblée en technique (ASIC) (circuit intégré dédié à une application) ou en technique FPGA (réseau de porte à champ programmable).
